# EUROPEAN PATENT SPECIFICATION

(11) **EP 2 449 384 B1**
(45) Date of publication and mention of the grant of the patent: **20.04.2016**
(21) Application number: 10726532.4
(22) Date of filing: 30.06.2010
(51) Int. Cl.: G01N 33/68, G01N 33/50

(54) **METHODS FOR PREDICTING CARDIOVASCULAR MORTALITY RISK**
VERFAHREN ZUR VORHERSAGE DES KARDIOVASKULÄREN STERBERISIKOS
Procédés pour la prédiction du risque de mortalité cardiovasculaire

(30) Priority: 01.07.2009 EP 09305635
(43) Date of publication of application: 09.05.2012
(73) Proprietor: INSERM - Institut National de la Santé et de la Recherche Médicale, 75013 Paris (FR); Hôpital Frédéric-Henri Manhès, 91700 Fleury Merogis (FR)
(72) Inventor: BOULANGER, Chantal, F-75015 Paris (FR); TEDGUI, Alain, F-75015 Paris (FR); LONDON, Gérard, F-75015 Paris (FR)
(74) Representative: Hirsch, Denise Marie
(86) International application number: PCT/EP2010/059295
(87) International publication number: WO 2011/000874

(56) References cited:
- WO-A1-2009/040133
- AMABILE N ET AL: "Circulating Endothelial Microparticles Are Associated with Vascular Dysfunction in Patients with End-Stage Renal Failure" JOURNAL OF THE AMERICAN SOCIETY OF NEPHROLOGY, WILLIAMS AND WILKINS, BALTIMORE, MD, US LNKD- DOI:10.1681/ASN.2005050535, vol. 16, no. 11, 1 November 2005 (2005-11-01), pages 3381-3388, XP002560429 ISSN: 1046-6673 [retrieved on 2005-09-28]
- SEO D ET AL: "Genomic medicine: bringing biomarkers to clinical medicine" CURRENT OPINION IN CHEMICAL BIOLOGY, CURRENT BIOLOGY LTD, LONDON, GB, vol. 9, no. 4, 1 August 2005 (2005-08-01), pages 381-386, XP004986468 ISSN: 1367-5931
- WANG ET AL: "Increased Circulating CD31+/CD42- Microparticles Are Associated With Impaired Systemic Artery Elasticity in Healthy Subjects" AMERICAN JOURNAL OF HYPERTENSION, ELSEVIER, vol. 20, no. 9, 29 August 2007 (2007-08-29), pages 957-964, XP022216995 ISSN: 0895-7061
- AMABILE NICOLAS ET AL: "Circulating Endothelial Microparticles: A Novel Biomarker for Prediction of Subsequent Death and Cardiovascular Events in End-stage Renal Disease" CIRCULATION, vol. 120, no. 18, Suppl. 2, November 2009 (2009-11), page S1010, XP002593249 & 82ND SCIENTIFIC SESSION OF THE AMERICAN-HEART-ASSOCIATION; ORLANDO, FL, USA; NOVEMBER 14 -18, 2009 ISSN: 0009-7322

## Description

### FIELD OF THE INVENTION

The present invention relates to a method for predicting cardiovascular mortality risk in a patient suffering from end stage renal disease.

### BACKGROUND OF THE INVENTION:

Cardiovascular diseases (CVDs) are the main cause of death in Europe, accounting for 49% of all deaths (and 30% of all premature deaths before the age of 65). Although age-specific mortality rates from CVDs have halved in western Europe in the last 20 years, the prevalence of CVD is actually increasing due to an ageing population. CVD is estimated to cost the European Union (EU) €169 billion annually.

Therefore there is a permanent need in the art for reliable biomarkers of cardiovascular mortality that will help physicians to identify patients at risk.

For example, cardiovascular disease is a major cause of death in patients with end stage renal disease (ESRD) and the projected life expectancy of these patients is much lower than that of the general population. Patients with ESRD have indeed a high prevalence of cardiovascular complications and arterial damage is a major contributor to their high cardiovascular mortality and morbidity. Vascular disease develops rapidly in uremic patients, involving endothelial dysfunction, a systemic disorder and a key variable in the pathogenesis of atherosclerosis and its complications. Mortality in patients with ESRD can be predicted by increases in inflammatory mediators, assymetric dimethylarginine plasma levels, hyperhomocysteinemia or arterial stiffness .

Endothelial dysfunction or activation results in a chronic inflammatory process accompanied by a loss of antithrombotic factors and an increase in vasoconstrictor and prothrombotic products, in addition to abnormal vasoreactivity. Endothelial dysfunction is associated and predicts increased rate of adverse cardiovascular events (Bonetti PO. et al. 2003; Zeiher AM. et al. 1991; McLenachan JM. et al. 1990; Zeiher AM. et al. 1991). Endothelial dysfunction in ESRD patients is associated with increased circulating levels of shed-membrane microparticles expressing endothelial cell specific markers (Amabile N. et al. 2005).

Microparticles (MPs) are plasma membrane vesicles shed from apoptotic or activated cell. They have been identified in plasma and in inflammatory tissues. Circulating MPs originate mostly from platelets, but MPs from other cell type, such as red blood cells, leukocytes, lymphocytes or endothelial cells, have been also identified in plasma (Amabile N. et al. 2005). Increases in circulating levels MPs of endothelial origin have been reported in several cardiovascular diseases and associate with the severity of endothelial dysfunction, supporting the concept that plasma level of endothelial microparticles is a surrogate marker of endothelial dysfunction in patients (Amabile N. et al. 2005; Mallat Z. et al. 2000; Koga H. et al. 2005; Werner N. et al. 2006)). The augmented release of endothelial MPs in ESRD could result from low endothelial shear stress, endothelial activation following chronic exposure to uremic toxins or increased oxidative stress (Boulanger CM. et al. 2007; Faure V. et al. 2001). Furthermore, circulating MPs from patients with coronary artery disease or with end-stage renal failure could also activate endothelial cells and impair endothelial NO release, therefore contributing to the general endothelial dysfunction observed in these patients (Amabile N. et al. 2005; Boulanger CM. et al. 2001).

WO2009/040133 discloses a method of assessing mortality risk caused by cardiovascular events, e.g. heart failure using osteopontin.

However determining whether circulating levels of endothelial MPs are predictive of clinical outcome or death has never been demonstrated.

### SUMMARY OF THE INVENTION:

The present invention relates to a method for predicting cardiovascular mortality risk in a patient, suffering from end stage renal disease, comprising determining the level of endothelial microparticles in a blood sample obtained from said patient.

### DETAILED DESCRIPTION OF THE INVENTION:

As arterial damage is a major contributor to cardiovascular mortality, the inventors examined whether or not increases in endothelial microparticles (EMPs) circulating levels could predict outcome in patients with end-stage renal disease (ESRD), who are at high cardiovascular risk. This prospective pilot study conducted in a Community Hospital (median followup: 50.5 months), included 81 stable hemodialyzed ESRD patients (59±14 yr; 63% male). Platelet-free plasma obtained 72 hrs after last dialysis was analyzed by flow cytometry analysis, and MPs cellular origin identified as endothelial (CD31+CD41-MPs; EMPs), platelets (CD31+CD41+MPs) or erythrocyte (CD235a+MPs). Main outcome measures were global and cardiovascular mortality (fatal myocardial infarction, stroke, acute pulmonary oedema and sudden cardiac death). Non-survivors (n=24) were older (p<0.001) and characterized by higher levels of EMPs (p<0.01) and hsCRP (p<0.05), and lower diastolic blood pressure (p<0.001). Patients with baseline EMP levels above median had a higher incidence of all-cause and cardiovascular death (p=0.0015). Baseline EMP levels independently predicted all-cause death (HR 1.28 [95% CI: 1.05-1.57] per 1000 EMPs/µL, p=0.016) and cardiovascular mortality (HR 1.38 [95% CI: 1.11-1.72], p=0.0035) after adjustment for confounding variables and was a stronger predictor of poor outcome than classical risk factors. This is the first direct evidence that increased plasma levels of endothelial microparticles is a robust independent predictor of severe cardiovascular outcome. Now in order to demonstrate that measure of circulating EMPs has valuable prognostic for cardiovascular mortality in the general population, the inventors test plasma levels of MPs of endothelial origin in 2000 subjects of the Framingham cohort 8th cycle (http://www.framinghamheartstudy.org/).

Therefore the present invention relates to a method for predicting cardiovascular mortality risk in a patient, suffering from end stage renal disease, comprising determining the level of endothelial microparticles in a biological sample obtained from said patient.

As used herein the term "endothelial microparticle" or "EMP" denotes a plasma membrane vesicle shed from an apoptotic or activated endothelial cell (Amabile N. et al. 2005). The size of endothelial microparticle ranges from 0,1 µm to 1µm in diameter. The surface markers of endothelial microparticles are the same as endothelial cells. Typically said surface markers include but are not limited to CD31, CD144, VE-Cadherin, and CD146. As endothelial cell, endothelial microparticles do not express specific surface markers such as CD41, CD4; CD14; CD235a; and CD11a. Therefore a typical endothelial microparticle is as CD31+CD41- microparticle.

The term "patient" as used herein denotes a mammal such as a rodent, a feline, a canine and a primate. Preferably, a patient according to the invention is a human.

Typically said patient may be previously diagnosed or not with a cardiovascular disease. The method according to the present invention can be supplied to a patient, which has been diagnosed as presenting one of the following coronary disorders:
- asymptomatic coronary artery coronary diseases with silent ischemia or without ischemia;
- chronic ischemic disorders without myocardial necrosis, such as stable or effort angina pectoris;
- acute ischemic disorders without myocardial necrosis, such as unstable angina pectoris;
- ischemic disorders with myocardial necrosis, such as ST segment elevation myocardial infarction or non-ST segment elevation myocardial infarction.
Accordingly said patient may suffer from a coronary disorder or vascular disorders selected from the group consisting of atherosclerotic vascular disease, such as aneurysm or stroke, asymptomatic coronary artery coronary diseases, chronic ischemic disorders without myocardial necrosis, such as stable or effort angina pectoris; acute ischemic disorders without myocardial necrosis, such as unstable angina pectoris; and ischemic disorders such as myocardial infarction.

Alternatively the patient may be asymptomatic for a coronary disorder or vascular disorder.

Furthermore, the patient is affected with a disease associated with a high risk of cardiovascular complications namely end stage renal disease. In these clinical conditions cardiovascular mortality is responsible for 40 to 50% of death, and the risk of death due to cardiovascular disease is 20 to 30 times higher than in general population.

The term "blood sample" means a whole blood, serum, or plasma sample obtained from the patient. Preferably the blood sample according to the invention is a plasma sample. A plasma sample may be obtained using methods well known in the art. For example, blood may be drawn from the patient following standard venipuncture procedure on tri-sodium citrate buffer. Plasma may then be obtained from the blood sample following standard procedures including but not limited to, centrifuging the blood sample at about 1,500*g for about 15-20 minutes (room temperature), followed by pipeting of the plasma layer. Platelet-free plasma (PFP) will be obtained following centrifugation at about 13,000*g for 5 min. In order to collect the endothelial microparticle, the plasma sample may be centrifuged in a range of from about 15,000 to about 20,000*g. Preferably, the plasma sample is ultra centrifuged at around 17,570*g at a temperature of about 4°C. Different buffers may be considered appropriate for resuspending the pelleted cellular debris which contains the endothelial microparticles. Such buffers include reagent grade (distilled or deionized) water and phosphate buffered saline (PBS) pH 7.4. Preferably, PBS buffer (Sheath fluid) is used. More preferably, the blood sample obtained from the patient is a platelet free platelet sample (PFP) sample. PFP may be separated from 10 ml citrated whole blood blood drawn from the fistula-free arm, 72 hours after the last dialysis. PFP may be obtained after citrate blood centrifugation at 1500g (15min), followed by 13000g centrifugation (5min, room temperature).

Standard methods for isolating endothelial microparticles are well known in the art. For example the methods may consist in collecting a population of microparticles from a patient and using differential binding partners directed against the specific surface markers of endothelial microparticles, wherein endothelial microparticles are bound by said binding partners to said surface markers.

The methods of the invention may comprise contacting the blood sample with a set of binding partners capable of selectively interacting with endothelial microparticles present in the blood sample. The binding partner may be an antibody that may be polyclonal or monoclonal, preferably monoclonal, directed against the specific surface marker of endothelial microparticles. In another embodiment, the binding partners may be a set of aptamers.

Polyclonal antibodies or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred.

Monoclonal antibodies or a fragment thereof can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985).

The binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods.

The binding partners such as antibodies or aptamers, may be labelled with a detectable molecule or substance, such as a fluorescent molecule, a radioactive molecule or any others labels known in the art. Labels are known in the art that generally provide (either directly or indirectly) a signal.

As used herein, the term "labelled", with regard to the antibody or aptamer, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody or aptamer, as well as indirect labelling of the probe or antibody by reactivity with a detectable substance. An antibody or aptamer of the invention may be labelled with a radioactive molecule by any method known in the art. For example radioactive molecules include but are not limited radioactive atom for scintigraphic studies such as I123, I124, In111, Re186, Re188.

Preferably, the antibodies against the surface markers are already conjugated to a fluorophore (e.g. FITC-conjugated and/or PE-conjugated). Examples include monoclonal anti-human CD62E-FITC, CDC105-FITC, CD51-FITC, CD106-PE, CD31-PE, and CD54-PE, available through Ancell Co. (Bayport, Minn.).

The aforementioned assays may involve the binding of the binding partners (ie. Antibodies or aptamers) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. The solid surfaces are preferably beads. Since endothelial microparticles have a diameter of roughly 0,1-1 µm, the beads for use in the present invention should have a diameter larger than 1µm. Beads may be made of different materials, including but not limited to glass, plastic, polystyrene, and acrylic. In addition, the beads are preferably fluorescently labelled. In a preferred embodiment, fluorescent beads are those contained in TruCount(TM) tubes, available from Becton Dickinson Biosciences, (San Jose, California).

According to the invention, methods of flow cytometry are preferred methods for determining the level of endothelial microparticles in the blood sample obtained from the patient. For example, fluorescence activated cell sorting (FACS) may be therefore used to separate in the blood sample the desired microparticles. Magnetic beads may be used to isolate endothelial microparticles (MACS).

For instance, beads labelled with monoclonal specific antibodies may be used for the positive selection of endothelial microparticles. Other methods can include the isolation of endothelial microparticles by depletion of non endothelial microparticles (negative selection). For example, endothelial microparticles may be excited with 488 nm light and logarithmic green and red fluorescences of FITC and PE may be measured through 530/30 nm and 585/42 nm bandpass filters, respectively. The absolute number of endothelial microparticles may then be calculated through specific software useful in practicing the methods of the present invention.

Typically, a fluorescence activated cell sorting (FACS) method such as described in Example 1 here below may be used to determining the levels of endothelial microparticles in the blood sample obtained from the patient.

Accordingly, the method of the invention comprises adding both labeled antibodies against surface markers that are specific to endothelial microparticles, putting said prepared sample into a container having a known number of solid surfaces wherein the solid surfaces are labeled with a fluorescent dye; performing a FACS flow cytometry on the prepared sample in order to calculate the absolute number of endothelial microparticles therein.

In one embodiment, the method of the invention may further comprise a step of comparing the level of endothelial microparticles with a predetermined value. As used herein, the term "predetermined value" refers to the levels of endothelial microparticles in the blood sample obtained from the general population or from a selected population of subjects. For example, the predetermined value may be of the level of endothelial microparticles obtained from patients who died from a cardiovascular disease. The predetermined value can be a threshold value, or a range. The predetermined value can be established based upon comparative measurements between patients who died from a cardiovascular disease and patients who survived with a cardiovascular disease. A differential between the level of endothelial microparticles determined by the method of the invention and the predetermined value is then indicative of a risk of cardiovascular mortality.

The method of the invention may be thus useful for classifying patient at risk for cardiovascular mortality and then may be used to choose the accurate treatment for said patient. Such a method may thus help the physician to make a choice on a therapeutic treatment. Costs of the treatments may therefore be adapted to risk of the patients.

A further object of the invention relates to method for monitoring the impact of a treatment administered to a patient suffering from end stage renal disease on cardiovascular mortality risk, comprising determining the level of endothelial microparticles in a blood sample obtained from said patient. More particularly, said method may further comprise a step consisting of a step of comparing the level of endothelial microparticles with a predetermined value. Said predetermined value may be the cardiovascular risk determined by the method of the invention before the treatment.

Typically, the treatment may consist in administering a therapeutically amount of an active ingredient selected from the group consisting of erythropoietin stimulating agents, or treatments associated with proven beneficial effects on endothelial dysfunction such as statins, angiotensin converting enzyme inhibitors or angiotensin receptor blockers. In another embodiment said treatment may consisting in reversing the endothelial dysfunction.

Another object of the invention relates to a method for predicting a risk of a major adverse cardiovascular events (MACE) in a patient, suffering from end-stage renal disease, comprising determining the level of endothelial microparticles in a blood sample obtained from said patient. Typically, MACE includes death, acute coronary syndromes, emergent percutaneous coronary intervention, stroke, congestive heart failure, chronic atrial fibrillation, and acute ischemia due to peripheral artery disease. In one embodiment, said may further comprise a step of comparing the level of endothelial microparticles with a predetermined value. The predetermined value can be established based upon comparative measurements between patients who had a MACE and patients who did not have a MACE. A differential between the level of endothelial microparticles determined by the method of the invention and the predetermined value is then indicative of a risk of a MACE.

The invention will be further illustrated by the following figures and examples. However, these examples and figures should not be interpreted in any way as limiting the scope of the present invention.

### FIGURES:

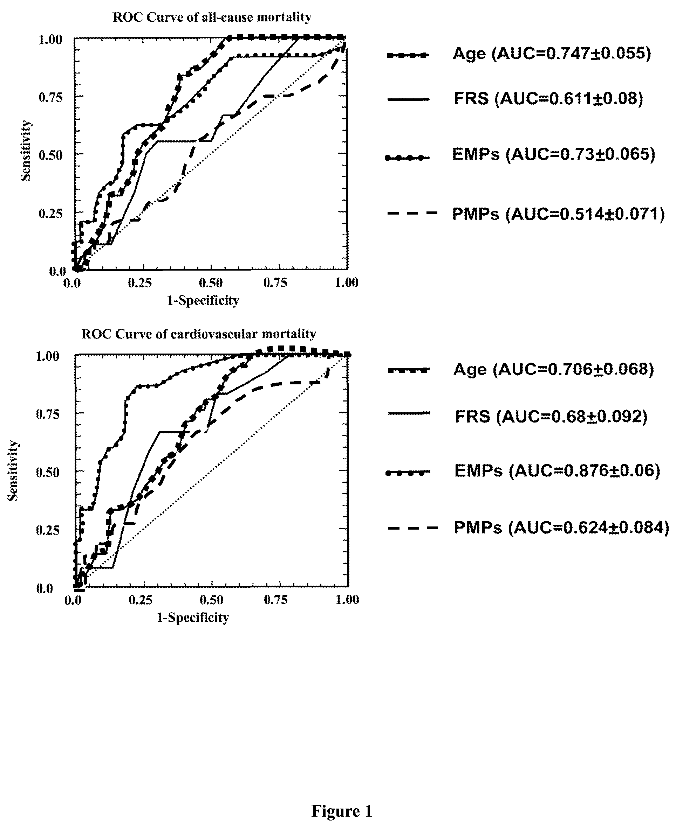

**Figure 1****: Receiver-operating characteristic curves of baseline MPs levels, using patients outcome as reference.** Whereas PMPs levels (green line) only predicted modestly global mortality (panel A) and cardiovascular mortality (panel B), baseline EMPs levels (red line) were strongly associated to outcome. EMPs levels were comparable to age (blue line) for global mortality, but demonstrated a larger AUC for cardiovascular mortality prediction.

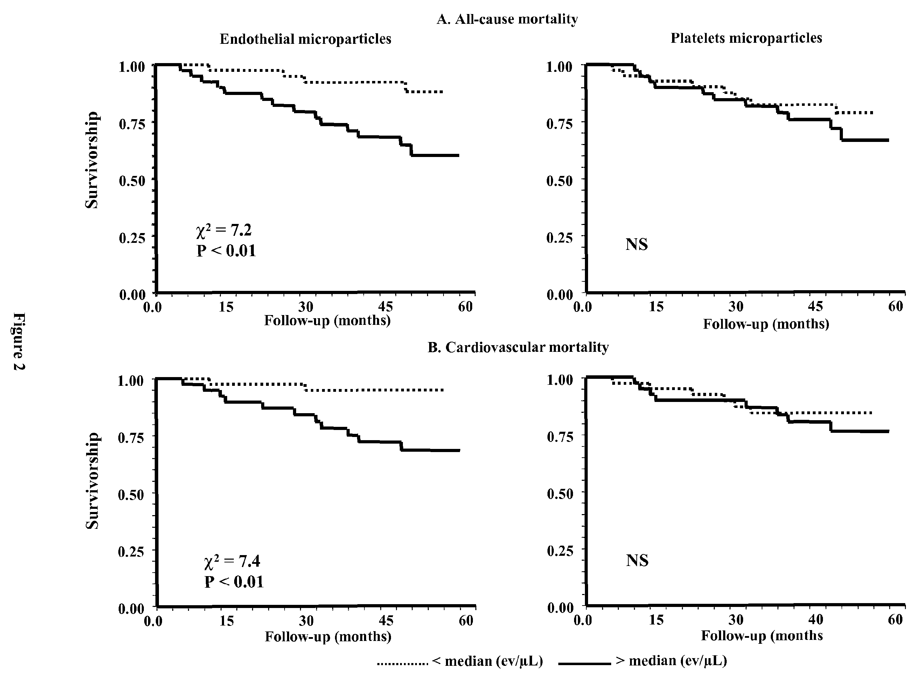

**Figure 2****: Kaplan-Meier estimates of survival free from global (panel A) and cardio-vascular mortality during follow up in patients with baseline EMPs and PMPs values below or over population median value.**

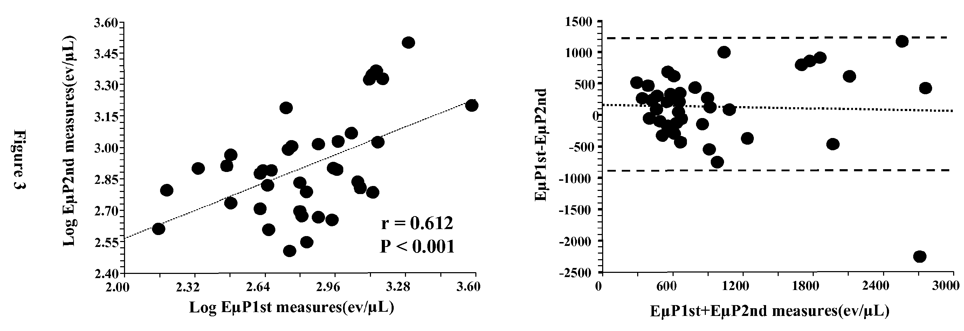

**Figure 3****: Reproducibility of EMPs measurement :** The reproducibility of EMPs measurement using 2 consequent measurements in n=41 patients. The median delay between measure #1 (EMP1) and measure #2 (EMP2) was 115 (3-296) days. The figure 1A depicts linear regression between log EMP1 and log EMP2 (the values of EMPs were log transformed because of their non-normal distribution among the sample) with a good correlation (Spearman coefficient =0.61, p<0.001). The figure 1B shows the Bland-Altman plot representation between the 2 measurements : each dot stands for one subject and the dashed lines represent the Mean +/- 1.96 standard deviation interval. The regression line showed no significant decreases or increases, suggesting good repeatability of the measures.

### EXAMPLE 1: PREDICTIVE VALUE OF CIRCULATING ENDOTHELIAL MICROPARTICLES FOR SUBSEQUENT DEATH IN END-STAGE RENAL DISEASE

### Material & Methods

**Patients:** We included 81 patients with ESRD from the Fleury-Mérogis hemodialysis center starting November 2003 till September 2008 **(Table 1).** Patients were eligible for inclusion when: (a) they were on haemodialysis therapy for ≥ 3 months (b) they had no clinical cardiovascular complication during the 6-month period preceding entry, and (c) they agreed to participate in the follow-up study, which was approved by our Institutional Review Board and adhered to the Declaration of Helsinski. Patients were dialyzed three times per week using high permeability membranes AN69 and Polysulfone. The duration of hemodialysis (HD) was individually tailored (4-6 h per session) to control body fluids and blood chemistries, and to achieve a Kt/V > 1.2 (1.46±0.13). The dialysate was prepared with double osmosis ultrapure water and delivered by a system including bicarbonate delivery, adjustable sodium concentration and controlled ultrafiltration. Patients were regularly prescribed iron and erythropoietin (darbepoietin). Thirty-five patients received adjunctive antihypertensive therapy (angiotensin-converting enzyme inhibitor and/or calcium channel blocker), n= 21 subjects were under betablockers and n=15 patients under statin therapy (atorvastatin 20 mg/day).

**Blood chemistries:** Blood chemistries, all determined on samples drawn prior to MPs measure, included **(Table 1):** hemoglobin, LDL-cholesterol, triglycerides, serum albumin, serum high-sensitive C-reactive protein (CRP), blood lipids, serum phosphates, Ca²⁺. Serum parathormone (iPTH 1-84) was measured by radioimmunoassay.

**Framingham risk scores (FRS) and European SCORE calculation:** Framingham sex specific equations were used to calculate FRS for general cardiovascular disease (D'Agostino RB, Sr., Vasan RS, Pencina MJ, et al. General cardiovascular risk profile for use in primary care: the Framingham Heart Study. Circulation 2008;117(6):743-53) over the 10 years. The European SCORE (estimation of the 10 years cardiovascular death risk) was calculated using the SCORE risk charts (Conroy RM, Pyorala K, Fitzgerald AP, et al. Estimation of ten-year risk of fatal cardiovascular disease in Europe: the SCORE project. Eur Heart J 2003;24(11):987-1003). Risks were calculated using systolic blood pressure, total and HDL-cholesterol, age, current smoking status, diabetes and use of antihypertensive medications.

**Microparticles analysis:** MP baseline levels and cellular origins were measured in platelet-free plasma (PFP) from stable patients. PFP was obtained from citrated whole blood drawn from the fistula-free arm, 72 hours after the last dialysis, following 1500g (15min) and 13000g (5min) centrifugations.

Flow cytometry analyses (EPICS XL, Beckman Coulter, France) were performed by two independent examiners unaware of the subject status (Amabile N. et al. 2005). PFP was incubated with fluorochrome-labeled antibodies (anti-CD31-Phycoerythrin (PE), anti-CD41-PC5 and anti-CD235a-Fluoroisothiacyanate (FITC) antibodies, Beckman Coulter France) or their respective isotypic immunoglobulins. MPs expressing phosphatidylserine were labeled using fluorescein-conjugated AnnexinV (Roche Diagnostics, France) with CaCl₂ (5mM). Events with a 0.1-1µm diameter were identified in forward scatter and side scatter intensity dot representation, and plotted on 1 or 2-colors fluorescence histograms. MPs were defined as elements with a size less than 1 µm and greater than 0.1 µm that were positively labeled by specific antibodies. Specific MP subpopulations were defined (Amabile N. et al. 2005): erythrocyte-derived (CD235a+), endothelium-derived (EMPs; CD 31+CD41-) and platelet MPs (PMPs; CD31+CD41+).

**Aortic Pulse wave velocity measurement:** Aortic pulse wave velocity (PWV) was measured in n=71 patients as described (Amabile N. et al. 2005).

**Clinical Endpoint assessment:** The primary outcome was the occurrence of any death during follow-up. Moreover, we recorded the number of fatal and non-fatal major adverse cardiovascular events (MACE; acute coronary syndromes, emergent percutaneous coronary intervention, stroke, congestive heart failure, chronic atrial fibrillation, acute ischemia due to peripheral artery disease).

**Statistical Analysis :** Data are expressed as median and range or mean ± standard deviation (SD) according to the normality of distribution. Quantitative variables with non-normal distribution were log-transformed to achieve normal distribution before correlations analysis. Mann Whitney and χ² tests were used for comparison of continuous and categorical variables among the population. The repeatability of EMP measurement was prospectively assessed in a subgroup of patients, using Spearman correlations and the Bland-Altman plot analysis (Bland JM, Altman DG. Statistical methods for assessing agreement between two methods of clinical measurement. Lancet 1986;1(8476):307-10).

The primary analyses concerned Cox proportional hazards model and the survival curves. Patients were censored the day of death or transplantation. Factors prognostic of survival were identified with the use of the univariable Cox proportional hazards regression model. The assumption of proportional hazards over the time and assumption of linearity was verified before the analyses and was met by all covariates. Due to high co-linearity between several variables, data reduction procedure was used to determine the final Cox model. The number of variables in the model was reduced using automatic backward stepwise selection algorithm. Variables with a significant association to outcome, as assessed by a p value<0.05, in univariate analysis were entered in the multivariate model. The variable with the strongest association was entered first, followed by the next strongest, until all variables related to the outcome are entered into the model. Any variable that has been entered but is no longer significant after other variables have been added to the model is sequentially deleted. Survival was estimated by the Kaplan-Meier product-limit method and compared by the Mantel (log-rank) test. Differences were considered significant at *p*<0.05. Receiver-operator characteristics (ROC) curve analyses were done to estimate the sensitivity and specificities. Statistical analysis was performed with NCSS 7.0 software (J. Hintze, Kaysville, Utah, USA).

### Results

**Patients Characteristics:** Table 1 shows the clinical and biochemical characteristics of the 81 patients included in the study. The follow-up averaged 50.5 [5-72] months and the median vintage was 40 months [3-324]. Statistical analysis of the repeatability of EMP determination (assessed in n=41 patients; mean delay of 115 (3-296) days between blood samples collection) revealed that EMP measures were reproducible with time (Figure 3).

During the follow-up period, 24 deaths (27%) were recorded, of which 17 were of cardiovascular causes (sudden cardiac death, n=5; acute pulmonary oedema, n=6; myocardial infarction, n=4; stroke, n=2). infarction, n=4; stroke, n=2). Furthermore, n=4 patients underwent kidney transplantation and were discharged from the study.

Deceased patients had greater levels of circulating endothelial MPs, lower diastolic blood pressure and were significantly older than survivors and had ab increase in hsCRP and serum calcium (Table 1). There was no difference regarding baseline cardiovascular risk factors, but moderate increases in Framingham score influenced by older age (Table 1).

**Outcome and prognostic impact of circulating MPs:** According to univariate Cox analysis for all-causes mortality, the significant covariates retained were age, diastolic blood pressure, history of cardiovascular diseases, FRS and EMP levels (Table 2). In multivariate Cox analyzes only age (HR=1.07 [95% CI: 1.03-1.11] per year; p<0.0001) and EMPs (HR=1.28 [95% CI: 1.05-1.57] per 1000 EMPs; p=0.014) were independent predictors of all-cause mortality.

Univariate Cox analysis showed that the significant covariates associated with cardiovascular mortality were age, body mass index, history of cardiovascular disease, diastolic blood pressure, hsCRP, Framingham score, and EMP levels (Table 2). In multivariate adjusted Cox model, age (HR=1.06 [95% CI: 1.01-1.12] per year; p<0.01), EMPs (HR=1.38 [95% CI: 1.11-1.72] per 1000 EMPs; p<0.005) and history of cardiovascular diseases (HR=5.36 (7 [95% CI: 1.17-24]) were the only independent predictors of cardiovascular mortality (Table 3). Comparable findings were observed in n=41 patients who had repeated measurement of EMPs where the analysis included the second measure of EMPs the follow-up corresponding to elapsed time from the second measure to end of follow-up. Comparable results demonstrate that EMPs was also an independent predictor of MACE (Table 4).

ROC curves were analyzed for specificity and sensitivity analyses of putative predictors of overall and cardiovascular mortality (Figure 1). The areas under the curve (AUC) were 75.6±7.5 (age), 84.2±6.3 (EMPs), 58.1±8.0 (PMPs), and 70.2±7.7 (FRS) for cardiovascular mortality. The optimal usable cut-off value of EMPs level was 1040 ev/ µL, with 87% sensitivity and 78% specificity for cardiovascular death prediction (positive predictive value=56%; negative predictive value=92%). Moreover, the optimal usable cut-off value of EMPs level was 1190 ev/ µL, with 63 % sensitivity and 78% specificity for cardiovascular death prediction (positive predictive value=59%; negative predictive value=81 %).

The cumulative events rates for composite cardiovascular and all-causes mortality are shown in Figure 2. The Kaplan-Meier curves for EMPs and PMPs, dichotomized for each of the medians, showed significant differences in cardiovascular and all-causes mortality for circulating EMPs (p=0.0015 and p=0.032, respectively; log rank test), but not for PMPs (Figure 2).

### Conclusions:

The present study performed in patients with ESRD and stable cardiovascular condition is the first demonstration that high plasma EMP levels are an independent and robust predictor of all-causes and cardiovascular mortality, whereas such potential was not revealed for MPs from other cell types or for the overall MP pool assessed by AnnexinV labeling.

We show here that circulating EMP levels are a robust predictor of all-cause and major adverse cardiovascular events in ESRD. This conclusion was not reached for plasma MPs from other cellular origin, or for annexinV+ MP. We also observed that hsCRP levels, previously reported as a predictor of all-cause and cardiovascular mortality in ESRD, were modestly but significantly increased in non-survivors when compared to survivors. However, multivariate analysis failed to demonstrate in the present study that hsCRP levels were associated with mortality, possibly because of the low number of subjects enrolled. The present data also show that diastolic blood pressure was lower in deceased ESRD patients when compared to survivors. This finding corroborates that of a previous study on a large ESRD population, indicating that low diastolic blood pressure was associated with increased death rate in ESRD patients, potentially by jeopardizing coronary perfusion. However, multivariate analysis failed to demonstrate a significant association between diastolic blood pressure and mortality in the 81 ESRD patients included in the present study. Nevertheless, despite the limited size of the sample, circulating EMPs appeared to be a more powerful independent predictor of adverse events in asymptomatic ESRD subjects than classical risk factors, Framingham risk score or PWV. Taken all together, the present results demonstrate that EMP measurement was reproducible, independently related to CV outcome, presented a stronger relationship to the predefined study endpoints than other risk factors in multivariate analysis and showed a larger AUC in ROC analysis than Framingham risk score. Therefore, circulating EMP measure fits with the recently proposed AHA guidelines criteria for evaluation of novel markers of cardiovascular risk and thus might represent a new independent tool for identification of subjects with a high profile risk among asymptomatic ESRD patients.

In conclusion, high levels of endothelial MPs are a strong independent predictor of cardiovascular events and all-causes mortality in hemodialized patients with ESRD. Detection of EMPs in human plasma could serve as an important tool in identifying asymptomatic patients at higher risk of developing cardiovascular diseases. The ability to identify these patients would lead to better risk stratification and more cost-effective preventive therapies. Finally, these findings lend support to the hypothesis that accumulation of circulating endothelial microparticles might be an important risk marker for cardiovascular disease in chronic renal failure.

**Table 1. Baseline clinical and biochemistry parameters (values are expressed as means±SD)**

| | All patients (n=81) | Deceased (n=24) | Alive (n=57) |
|---|---|---|---|
| Age (yrs) | 58.7±14 | 70.6±9.2 | 55.1±13.2*** |
| Sex, M/F ratio | 51/30 | 14/10 | 37/20 |
| Diabetes, n | 8 | 4 | 4 |
| History of cardiovascular diseases (n) | 39 | 18 | 213 |
| Vintage (months) | 63±64 | 70±60 | 58±66 |
| BMI (kg/m²) | 25.5±5.1 | 26.6±6.0 | 25.3±4.9 |
| Smoking (packs.year) | 7.9±15.8 | 9.1±19.8 | 7.2±13.3 |
| Current smoker | 2 | 0 | 2 |
| Systolic BP (mm Hg) | 139.4±21.3 | 132.4±20.1 | 142±21.2 |
| Diastolic BP (mm Hg) | 77.7±12.5 | 70.6±10.5 | 80.7±12.4** |
| | | | |
| Total Cholesterol (mMol/L) | 4.28±1.10 | 4.50±1.10 | 4.20±1.15 |
| hsCRP (mg/L) | 5.4±4.3 | 7.2±4.7 | 4.7±4.0* |
| Serum albumin (g/L) | 37.0±2.9 | 35.2±3.1 | 37.7±2.7 |
| Hemoglobin (g/dL) | 11.2±1.4 | 11.1±1.2 | 11.2±1.5 |
| | | | |
| FRS for General cardiovascular disease | 13.14±4.68 | 15.5±3.36 | 12.6±5.1 |
| European SCORE | 2.33±2.22 | 2.78±1.80 | 2.2±2.3 |
| Parathormone (pg/mL) | 332±271 | 314±305 | 338±261 |
| Circulating | | | |
| Microparticles levels : | | | |
| *Endothelial MPs (ev*/µ*L)* | 1142±1020 | 1959±1925 | 1034±915*** |
| *Platelets MPs (ev*/*µL)* | 4087±3530 | 4260±36411 | 4014±3512 |
| | | | |
| Medication: | | | |
| | | | |
| ACE inhibitors, (n) | 153 | 2 | 13 |
| Statins, (n) | 25 | 14 | 11 |
| Beta Blockers (n) | 21 | 5 | 16 |

| | | | |
|---|---|---|---|
| Abbreviations used: BMI - body mass index; BP - blood pressure; hsCRP - high sensitive C-reactive protein; Deceased vs. alive * - P<0.05; ** P<0.01; *** P<0.001 | | | |

**Table 2. Univariate (A) and multivariate (B) Cox model for all-cause mortality in ESRD patients (Z value is significant for all values above 1.96; HR stands for hazard ratio).**

| A. Univariate analysis | | |
|---|---|---|
| Variable | **P-value** | **HR [95% CI]** |
| **Age (yrs)** | **<0.0001** | **1.08 (1.04-1.12)** |
| Gender (0-male;1-female) | 0.431 | 0.70 (0.32-1.61) |
| Body mass index (kg/m²) | 0.292 | 1.04 (0.96-1.13) |
| Diabetes | 0.539 | 0.70 (0.23-2.09) |
| *History of CV diseases* | **0.0024** | **4.21 (1.67-110.65)** |
| Systolic blood pressure (mm Hg) | 0.130 | 0.98 (0.96-1.01) |
| **Diastolic blood pressure (mm Hg)** | **<0.001** | **0.93 (0.89-0.97)** |
| Total cholesterol (mMol/L) | 0.407 | 1.17 (0.81-1.68) |
| HsCRP (mg/L) | 0.055 | 3.05 (0.98-9.56) |
| *FRS (per unit)* | **0.017** | **1.11 (1.02-1.21)** |
| European SCORE (per unit) | 0.359 | 1.07 (0.93-1.23)- |
| Serum Albumin (g/L) | 0.090 | 0.87 (0.74-1.02) |
| Hemoglobin (g/L) | 0.496 | 0.91(0.68-1.20) |
| Serum phosphates (mmol/l)) | 0.0.404 | 2.02 (0.39-10.61) |
| **Endothelial MPs (1000ev/µL)** | **0.0018** | **1.37 (1.13.1-1.68)** |
| Platelets MPs (1000ev/µL) | 0.720 | 1.01 (0.91-1.14) |
| Smoking (packs/yr) | 0.428 | 1.01 (0.98-1.03) |

| B. Multivariate analysis | | |
|---|---|---|
| **Variable** | **P-value** | **HR [95% CI]** |
| **Age (yrs)** | **<0.001** | **1.07 (1.03-1.11)** |
| **Endothelial MPs (1000ev/µL)** | **0.0140** | **1.28 (1.05-1.57)** |
| **Diastolic blood pressure (per mHg)** | **0.020** | **0.94 (0.90-0.99)** |
| History of CV diseases | 0.070 | 0.41(0.15-1.08) |
| FRS (per unit) | 0.404 | 0.94(0.82-1.08) |
| **R² for model: 0.323, P<0.001** | | |

**Table 3. Univariate (A) and multivariate (B) Cox models for cardiovascular mortality in ESRD patients (Z value is significant for all values above 1.96; HR stands for hazard ratio).**

| A. Univariate Analysis | | |
|---|---|---|
| **Variable** | **P-value** | **HR [95% CI]** |
| **Age (yrs)** | **< 0.001** | **1.08 (1.03-1.13)** |
| Gender (0-male;1-female) | 0.498 | 0.70 (0.26-1.94) |
| **Body mass index (kg/m²)** | **0.039** | **1.10 (1.01-1.20)** |
| Diabetes | 0.468 | 0.62 (0.17-2.24) |
| **History of CV diseases** | **0.004** | **19.7 (2.60-151.0)** |
| Systolic blood pressure (mm Hg) | 0.854 | 1.00 (0.97-1.02) |
| Diastolic blood pressure (mm Hg) | **0.013** | **0.95 (0.91-1.00)** |
| Total cholesterol (mMol/L) | 0.504 | 1.17 (0.74-1.85) |
| **HsCRP (mg/L)** | **0.017** | **1.12 (1.02-1.24)** |
| **FRS (per unit)** | **0.011** | **1.16 (1.04-1.31)** |
| European SCORE (per unit) | 0.261 | 1.09 (0.93-1.28) |
| Serum Albumin (g/L) | 0.267 | 0.89 (0.72-1.09) |
| Hemoglobin (g/L) | 0.951 | 1.00 (0.70-1.43) |
| Serum phosphates (mmol/l) | 0.665 | 1.58 (0.20-12.9) |
| **Endothelial MPs (per 1000ev/µL)** | **<0.0001** | **1.54 (1.25-1.90)** |
| Platelets MPs (per 1000ev/µL) | 0.238 | 1.07 (0.96-1.20) |
| Smoking (packs/yr) | 0.395 | 1.01 (0.93-1.04) |

| B. Multivariate analysis: Cardiovascular mortality | | |
|---|---|---|
| **Variable** | **P-value** | **HR [95% CI]** |
| **Endothelial MPs (per 1000ev/µL)** | **0.0035** | **1.38 (1.11-1.72)** |
| **Age (yrs)** | **0.0120** | **1.06 (1.01-1.12)** |
| **History of CV disease** | **0.031** | **5.36 (1.17-24.7)** |
| Diastolic BP | 0.108 | 0.95 (0.90-1.01) |
| HsCRP (mg/l) | 0.380 | 1.06 (0.93-1.22) |
| BMI (kg/m²) | 0.778 | 1.02 (0.91-1.13) |
| Framingham score (per unit) | 0.832 | 1.03 (0.79-1.35) |
| **R² for model: 0.318, p<0.0001** | | |

**Table 4: Univariate (A) and multivariate (B) Cox models for MACE in ESRD patients (Z value is significant for all values above 1.96; HR stands for hazard ratio).**

| A. Univariate Analysis | | | |
|---|---|---|---|
| | MACE | | |
| Variable | Z - Wald | P-value | HR [95% CI] |
| Age (yrs) | 4.46 | <0.0001 | 1.07 (1.04-1.11) |
| Gender | 0.19 | NS | - |
| Body mass index (kg/m²) | 1.75 | NS | - |
| Diabetes | 2.08 | 0.038 | 2.28 (1.05-4.96) |
| History of CV diseases | 4.47 | <0.0001 | 26.3 (6.3-110.1) |
| Systolic blood pressure | 1.00 | NS | - |
| (mm Hg) | | | |
| Diastolic blood pressure (mm Hg) | -1.99 | 0.046 | 0.97 (0.93-1.00) |
| Pulse Pressure (mm Hg) | 2.32 | 0.020 | 1.02 (1.0-1.04) |
| Total cholesterol (mMol/L) | 0.61 | NS | - |
| HDL cholesterol (mMol/L) | -0.82 | NS | - |
| Triglyceride (mMol/L) | 1.71 | NS | - |
| HsCRP (mg/L) | 1.14 | NS | - |
| FRS | 3.47 | 0.0003 | 1.16 (1.07-1.26) |
| European SCORE | 3.01 | 0.0026 | 1.16 (1.06-1.27) |
| Serum Albumin (g/L) | -1.06 | NS | - |
| Hemoglobin (g/L) | -1.20 | NS | - |
| Parathormone (pg/mL) | -3.41 | 0.0006 | 0.20 (0.08-0.50) |
| Serum Calcium (mMol/L) | -0.31 | NS | - |
| Serum Phosphates (mMol/L) | 0.02 | NS | - |
| LogEndothelial MPs (ev/µL) | 3.66 | 0.0003 | 6.53 (2.39-17.8) |
| LogPlatelets MPs (ev/µL) | 0.95 | NS | - |
| LogAnnexin V+ MPs (ev/µL) | 0.83 | NS | - |
| LogErythrocyte MPs (ev/µL) Medications: | -0.19 | NS | - |
| ACE inhibitors | 0.31 | NS | - |
| Statins | -1.1 | NS | - |
| Darbepoietin, (µg/week) | 3.24 | 0.0012 | 1.03 (1.01-1.04) |
| Beta Blockers | -1.2 | NS | - |

| B. Multivariate Analysis: MACE | | | |
|---|---|---|---|
| Model 1 (R² for model: 0.55, p<0.001) | | | |
| Variable | Z - Wald | P-value | HR [95% CI] |
| History of cardiovascular diseases | 3.90 | 0.0001 | 18.3 (4.20-78.9) |
| Age (yrs) | 3.14 | 0.0014 | 1.06 (1.02-1.10) |
| Log Endothelial MPs (ev/µL) | 2.33 | 0.0018 | 3.28 (1.21-8.89) |
| *The following variables did not reach statistical significance: Darbepoietin treatment, Parathormone level, Diabetes, Pulse pressure, Diastolic blood pressure* | | | |
| Model 2 (R² for model: 0.52, p<0.001) | | | |
| Variable | Z - Wald | P-value | HR [95% CI] |
| History of cardiovascular disease | 4.18 | <0.0001 | 22.3 (5.20-95.5) |
| Darbepoietin treatment (µg/week) | 2.80 | 0.0051 | 1.02 (1.01-1.05) |
| Log Endothelial MPs (ev/µL) | 2.64 | 0.0084 | 3.91 (1.42-10.8) |
| *The following variables did not reach statistical significance: FRS, Parathormone level*. *Pulse pressure, Diastolic blood pressure* | | | |
| Model 3 (R² for model: 0.52, p<0.001) | | | |
| Variable | Z - Wald | P-value | HR [95% CI] |
| History of cardiovascular diseases | 4.18 | <0.0001 | 22.3 (5.2-95.5) |
| Darbepoietin treatment | 2.80 | 0.0051 | 1.02 (1.01-1.05) |
| Log Endothelial MPs (ev/µL) | 2.64 | 0.0084 | 3.91 (1.42-10.8) |
| *The following variables did not reach statistical significance: European SCORE, Parathormone level. Diabetes, Pulse Pressure, Diastolic blood pressure* | | | |

### EXAMPLE 2: PREDICTIVE VALUE OF CIRCULATING ENDOTHELIAL MICROPARTICLES FOR SUBSEQUENT DEATH IN THE GENERAL POPULATION

In order to further demonstrate that measure of circulating EMPs has valuable prognostic for cardiovascular mortality in the general population, the inventors test plasma levels of MPs of endothelial origin in 2000 subjects of the Framingham cohort 8th cycle (http://www.framinghamheartstudy.org/).

**Outcome:** The outcomes of interest are incidence of a first cardiovascular event and all-cause mortality during follow-up. Major CVD events include fatal or nonfatal coronary heart disease (myocardial infarction, coronary insufficiency, and angina pectoris), stroke or transient ischemic attack, intermittent claudication, or heart failure. Criteria for these events have been described earlier (Bland JM, Altman DG. Statistical methods for assessing agreement between two methods of clinical measurement. Lancet 1986;1:307-10.). The outcome is expected to reach 150 major cardiovascular events for the 2000 subjects included.

**Statistical Analysis:** Data are expressed as median and range or mean±SD according to the normality of distribution. Quantitative variables with non-normal distribution are log-transformed to achieve normal distribution before correlations analysis. Mann-Whitney and χ² tests are used for comparison of continuous and categorical variables among the population. The primary analyses concerns Cox proportional hazards model and the survival curves. Patients are censored the day of death or occurrence of major events. Prognostic factors of survival are identified with the use of the univariable Cox proportional hazards regression model. The assumption of proportional hazards over the time and assumption of linearity is verified before the analyses for all covariates. Due to possible high co-linearity between several variables, data reduction procedure might be used to determine the final Cox model. The number of variables in the model are reduced using automatic backward stepwise selection algorithm. Variables with a significant association to outcome, as assessed by a p value<0.05 in univariate analysis, are entered in the multivariate model. The variable with the strongest association is entered first, followed by the next strongest, until all variables related to the outcome are entered into the model. Any variable that has been entered but is no longer significant after other variables have been added to the model is sequentially deleted. Survival is estimated by the Kaplan-Meier product-limit method and compared by the Mantel (log-rank) test. Differences are considered significant at *p*<0.05. Receiver-operator characteristics (ROC) curve analyses are done to estimate the sensitivity and specificities.

### REFERENCES:

Amabile N, Guerin AP, Leroyer A, et al. Circulating endothelial microparticles are associated with vascular dysfunction in patients with end-stage renal failure. J Am Soc Nephrol 2005;16(11):3381-8.
Bernal-Mizrachi L, Jy W, Jimenez JJ, et al. High levels of circulating endothelial microparticles in patients with acute coronary syndromes. Am Heart J 2003;145(6):962-70.
Bonetti PO, Lerman LO, Lerman A. Endothelial dysfunction: a marker of atherosclerotic risk. Arterioscler Thromb Vasc Biol 2003;23(2):168-75.
Boulanger CM, Amabile N, Guerin AP, et al. In vivo shear stress determines circulating levels of endothelial microparticles in end-stage renal disease. Hypertension 2007;49(4):902-8.
Boulanger CM, Scoazec A, Ebrahimian T, et al. Circulating microparticles from patients with myocardial infarction cause endothelial dysfunction. Circulation 2001;104(22):2649-52.
Combes V, Simon AC, Grau GE, et al. In vitro generation of endothelial microparticles and possible prothrombotic activity in patients with lupus anticoagulant. J Clin Invest 1999;104(1):93-102.
Faure V, Dou L, Sabatier F, et al. Elevation of circulating endothelial microparticles in patients with chronic renal failure. J Thromb Haemost 2006;4(3):566-73.
Koga H, Sugiyama S, Kugiyama K, et al. Elevated levels of VE-cadherin-positive endothelial microparticles in patients with type 2 diabetes mellitus and coronary artery disease. J Am Coll Cardiol 2005;45(10):1622-30.
Mallat Z, Benamer H, Hugel B, et al. Elevated levels of shed membrane microparticles with procoagulant potential in the peripheral circulating blood of patients with acute coronary syndromes. Circulation 2000;101(8):841-3.
McLenachan JM, Vita J, Fish DR, et al. Early evidence of endothelial vasodilator dysfunction at coronary branch points. Circulation 1990;82(4):1169-73.
VanWijk MJ, Nieuwland R, Boer K, van der Post JA, VanBavel E, Sturk A. Microparticle subpopulations are increased in preeclampsia: possible involvement in vascular dysfunction? Am J Obstet Gynecol 2002;187(2):450-6.
Werner N, Wassmann S, Ahlers P, Kosiol S, Nickenig G. Circulating CD31+/annexin V+ apoptotic microparticles correlate with coronary endothelial function in patients with coronary artery disease. Arterioscler Thromb Vasc Biol 2006;26(1):112-6.
Zeiher AM, Drexler H, Wollschlager H, Just H. Endothelial dysfunction of the coronary microvasculature is associated with coronary blood flow regulation in patients with early atherosclerosis. Circulation 1991;84(5):1984-92.
Zeiher AM, Drexler H, Wollschlager H, Just H. Modulation of coronary vasomotor tone in humans. Progressive endothelial dysfunction with different early stages of coronary atherosclerosis. Circulation 1991;83(2):391-401.

## Claims

1. A method for predicting cardiovascular mortality risk in a patient suffering from end stage renal disease, comprising determining the level of endothelial microparticles in a blood sample obtained from said patient and comparing said level of endothelial microparticles with a predetermined value.

2. The method according to claim 1 wherein said blood sample is plasma sample.

3. A method for monitoring the impact of a treatment administered to a patient suffering from end stage renal disease on cardiovascular mortality risk, comprising determining the level of endothelial microparticles in a blood sample obtained from said patient.

4. The method according to claim 3, wherein said method further comprises a step consisting of a step of comparing the level of endothelial microparticles with a predetermined value.

5. The method according to claim 4, wherein said predetermined value is the cardiovascular risk determined by the method according to any of claims 1 to 2 before the treatment.

6. A method for predicting a major adverse cardiovascular event (MACE) in a patient suffering from end stage renal disease, comprising determining the level of endothelial microparticles in a blood sample obtained from said patient.

7. The method according to claim 6 wherein said method further comprises a step of comparing the level of endothelial microparticles with a predetermined value.

8. The method according to claims 6 to 7 wherein said MACE is selected from the group consisting of death, acute coronary syndromes, emergent percutaneous coronary intervention, stroke, congestive heart failure, chronic atrial fibrillation, and acute ischemia due to peripheral artery disease.

## Patentansprüche

1. Eine Methode zur Voraussage der kardiovaskulären Mortalität bei einem Patienten, der an einer Nierenerkrankung im Endstadium leidet, umfassend die Determinierung des Pegels an endothelialen Mikropartikeln in einer von besagtem Patienten stammenden Blutprobe und den Vergleich besagten Pegels endothelialer Mikropartikel mit einem vorgegebenen Wert.

2. Die Methode gemäß Anspruch 1, **dadurch gekennzeichnet, dass** die besagte Blutprobe eine Plasmaprobe ist.

3. Eine Methode zur Überwachung des Einflusses einer Behandlung in Bezug auf ein kardiovaskuläres Mortalitätsrisiko, die einem Patienten administriert wird, der an einer Nierenerkrankung im Endstadium leidet, umfassend die Determinierung des Pegels an endothelialen Mikropartikeln in einer von besagtem Patienten stammenden Blutprobe.

4. Die Methode gemäß Anspruch 3, **dadurch gekennzeichnet, dass** besagte Methode darüber hinaus einen Schritt umfasst, der den Pegel an endothelialen Mikropartikeln mit einem vorgegebenen Wert vergleicht.

5. Die Methode gemäß Anspruch 4, **dadurch gekennzeichnet, dass** der vorgegebene Wert das kardiovaskuläre Risiko darstellt, welches durch die Methode aus einem beliebigen der Ansprüche 1 bis 2 vor der Behandlung determiniert wird.

6. Eine Methode zur Vorhersage eines MACE (schwerwiegender kardiovaskulärer Nebeneffekt) bei einem Patienten, der an einer Nierenerkrankung im Endstadium leidet, umfassend die Determinierung des Pegels an endothelialen Mikropartikeln in einer von besagtem Patienten stammenden Blutprobe.

7. Die Methode gemäß Anspruch 6, **dadurch gekennzeichnet, dass** besagte Methode darüber hinaus einen Schritt umfasst, der den Pegel an endothelialen Mikropartikeln mit einem vorgegebenen Wert vergleicht.

8. Die Methode gemäß Ansprüche 6 bis 7, **dadurch gekennzeichnet, dass** besagter MACE aus einer Gruppe ausgewählt wird, die folgendes umfasst: Tod, akute Koronarsyndrome, notfällige perkutane Koronarintervention, Schlaganfall, kongestive Herzinsuffizienz, chronisches Vorhofflimmern, und akute Ischämie auf Grund von peripheren Arterienerkrankungen

## Revendications

1. Procédé pour prévoir le risque de mortalité cardiovasculaire chez un patient souffrant d'une maladie rénale au stade final, comprenant la détermination du niveau de microparticules endothéliales dans un échantillon de sang obtenu auprès dudit patient et la comparaison dudit niveau de microparticules endothéliales à une valeur prédéterminée.

2. Procédé selon la revendication 1, dans lequel ledit échantillon de sang est un échantillon de plasma.

3. Procédé pour contrôler l'impact d'un traitement administré à un patient souffrant d'une maladie rénale au stade final sur un risque de mortalité cardiovasculaire, comprenant la détermination du niveau de microparticules endothéliales dans un échantillon de sang obtenu auprès dudit patient.

4. Procédé selon la revendication 3, dans lequel ledit procédé comprend en outre une étape constituée par une étape de comparaison du niveau de microparticules endothéliales à une valeur prédéterminée.

5. Procédé selon la revendication 4, dans lequel ladite valeur prédéterminée est le risque cardiovasculaire déterminé par le procédé selon n'importe laquelle des revendications 1 à 2 avant le traitement.

6. Procédé pour prévoir un événement cardiovasculaire défavorable majeur (MACE) chez un patient souffrant d'une maladie rénale au stade final, comprenant la détermination du niveau de microparticules endothéliales dans un échantillon de sang obtenu auprès dudit patient.

7. Procédé selon la revendication 6, dans lequel ledit procédé comprend en outre une étape de comparaison du niveau de microparticules endothéliales à une valeur prédéterminée.

8. Procédé selon les revendications 6 à 7, dans lequel ledit MACE est sélectionné à partir du groupe constitué par la mort, des syndromes coronaires aigus, une intervention coronaire percutanée émergente, un accident vasculaire cérébral, une insuffisance cardiaque congestive, une fibrillation atriale chronique et une ischémie aiguë en raison de la maladie d'artère périphérique.
